Europäisches Patentamt

**European Patent Office** (11) Numéro de publication: **0 174 226**

Office européen des brevets **B1**

(12) **FASCICULE DE BREVET EUROPEEN**

(45) Date de publication du fascicule du brevet: **23.01.91**

(21) Numéro de dépôt: **85401545.0**

(22) Date de dépôt: **26.07.85**

(51) Int. Cl.⁵: **C 12 Q 1/68, C 12 P 19/34 //
(C12Q1/68, C12R1:01)**

(54) Sonde et procédé pour la détection de micro-organismes détérminés, notamment des legionella dans les milieux les contenant.

(30) Priorité: **02.08.84 FR 8412250**

(43) Date de publication de la demande:
**12.03.86 Bulletin 86/11**

(45) Mention de la délivrance du brevet:
**23.01.91 Bulletin 91/04**

(84) Etats contractants désignés:
**AT BE CH DE FR GB IT LI LU NL SE**

(56) Documents cités:
**EP-A-0 120 658
EP-A-0 133 288
WO-A-84/02721
CHEMICAL ABSTRACTS, vol. 99, no. 15, 10
octobre 1983, page 350, no. 118946k, Columbus,
Ohio, US; W. LUDWIG et al.: "A phylogenetic
analysis of Legionella"
CHEMICAL ABSTRACTS, vol. 101, no. 17, 22
octobre 1984, page 363, no. 147436j, Columbus,
Ohio, US; R.J. VAN KETEL et al.: "Molecular
epidemiology of Legionella pneumophila
serogroup I"**

(73) Titulaire: **INSTITUT NATIONAL DE LA SANTE ET
DE LA RECHERCHE MEDICALE (INSERM)
101, rue de Tolbiac
F-75654 Paris Cédex 13 (FR)**
(73) Titulaire: **INSTITUT PASTEUR
28, rue du Docteur Roux
F-75715 Paris Cedex 15 (FR)**

(72) Inventeur: **Tchen, Paul
18, rue du Télégraphe
F-92000 Nanterre (FR)**
Inventeur: **Desplaces, Nicole
8, rue Dombasles
F-75015 Paris (FR)**
Inventeur: **Grimont, Patrick
207, rue de Vaugirard
F-75015 Paris (FR)**
Inventeur: **Grimont, née Besse Francine
207, rue de Vaugirard
F-75015 Paris (FR)**

(74) Mandataire: **Gutmann, Ernest et al
S.C. Ernest Gutmann - Yves Plasseraud 67,
boulevard Haussmann
F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP  0 174 226  B1**

(56) Documents cités:
CHEMICAL ABSTRACTS, vol. 101, no. 15, 8 octobre 1984, page 360, no. 126407k, Columbus, Ohio, US; S.E. HOFFNER et al.: "Characterization of Swedish isolates of Legionella pneumophila by means of capillary gas chromatography and DNA/DNA-hybridization studies"

**Description**

*Legionella pneumophila* (L.P.) responsable de la maladie des légionnaires (M.L.) a été cliniquement individualisée pendant l'été 1976. Elle est l'agent principal des légionelloses. Le terme "legionellose" est utilisé pour nommer les infections causées par les *Legionellaceae.* Toutes ces bactéries, mais surtout L.P., ont été mises en évidence dans und grand nombre de prévelèvements d'eau provenant de lacs, de rivières, de tours aérorefroidissantes et principalement de réseaux de distribution d'eau — ces deux derniers étant à l'origine de cas épidémiques et sporadiques de maladie des légionnaires — aussi bien en Amérique du Nord qu'en Europe.

Les caractéristiques taxonomiques et phénotypiques des *Legionellaceae* sont bien connues. Elles sont décrites par BRENNER D.J., FEELEY J.C., et WEAVER R.E., dans le BERGEY'S MANUAL OF SYSTEMATIC BACTERIOLOGY, Vol. I, 1984, Williams and Wilkins Ed. Baltimore/London. On mentionnera les caractères que ces bactéries ont en commun:

il s'agit de bacilles gram négatifs aérobies stricts,

exigeants en besoins nutritifs complexes pour leur culture au laboratoire:principalement dépendants en L. cystéine HCL et en sels ferriques. L'oxydase est faiblement positive, les nitrates ne sont pas réduits, l'uréase est négative, la gélatine est liquéfiée. La paroi comporte des chaînes d'acides gras ramifiés.

Parmi les *Legionellaceae,* l'espèce dénommée *Legionella pneumophila* présente les mêmes caractères phénotypiques des *Legionellaceae.* L'identification d'espèce repose sur l'utilisation d'immuns sérums fluorescents spécifiques anti-*Legionella* polyvalents (8 sérogroupes disponibles actuellement), puis monovalents, en immunofluorescence directe.

Le diagnostic *in vitro* de la M.L., tel qu'il est pratiqué à l'heure actuelle, repose sur la mise en évidence de *Legionella* en culture ou par l'ascension significative du taux des anticorps. La recherche de *Legionella* par culture dans des prélèvements pathologiques (tissus pulmonaires, exudats trachéaux, expectoration, liquide pleureal, sang, etc.) est délicate et nécessite des milieux de culture gélosés spéciaux connus sous l'expression anglaise "Buffered Charcoal Yeast Extract" (milieu tamponné contenant du charbon, de l'extrait de levure, du pyrophosphate ferrique et de la L-cystéine HCl) (B C Y E, décret par PASCUILLE A.W. et coll. J. Infect, Dis., 1980, 141, 727—732) rendus partiellement sélectifs par l'addition d'antibiotiques, par exemples tels que décrits par EDELSTEIN P.H., J. Clin. Microbiol., 1981, 14, 298—303. Elle implique aussi des opérations de décontamination préalables ou simultanées:traitement par la chaleur à 60°C pendant 1 à 2 minutes, ou en milieu tamponné acide à pH 2,2 pendant 10 minutes. De tels traitements, qui ont pour vocation de renforcer la sélectivité des épreuves de diagnostic, présentent en même temps l'inconvénient de diminuer la quantité de *Legionella* dans tout prélèvement traité de cette façon. En outre la croissance de *L. pneumophila* est lente et les premières colonies n'apparaissent que 2 à 4 jours après l'ensemencement. L'identification de ces colonies repose sur des critères liés à des conditions de culture (dépendance vis-à-vis de la L. cystéine) ou à des caractéres biochimiques et antigéniques; ces derniers sont détectés en immunofluorescence directe par des antisérums spécifiques de chaque sérogroupe de *L. pneumophila.*

Ces recherches, déjà longues et fastidieuses lorsqu'elles sont appliquées à des prélèvements pathologiques susceptibles de contenir des L.P. à des concentrations relativement élevées, deviennent pratiquement impossibles à faire, dès lors qu'elles seraient appliquées à l'étude et au contrôle des contaminations ou de la pollution des eaux circulants ou de façon plus générale de l'environnement. Las lenteur de la croissance des légionelles, la présence d'autres microorganismes contaminants à développement plus rapide, la difficulté de mettre au point des milieux réellement sélectifs rendent des contrôles en routine particulièrements difficiles. A ces difficultés s'ajoutent d'ailleurs encore la diversité qui peut être observée au niveau des propriétés immunologiques des différentes espèces de légionelles.

L'utilisation de techniques mettant en oeuvre l'hybridation d'une séquence nucléotidique particulière clonée, notamment d'un gène chromosomique cloné, avec les génomes, préablement rendus accessibles à une hybridation, des microorganismes recherchés, ne pouvait guère a priori être envisagée. Une première raison en était le fait que l'on ne dispose pas à ce jour de séquences d'ADN suffisamment spécifiques pour que cette méthode puisse être mise en oeuvre avec une certitude suffisante que l'hybridation sera réellement sélective. Cette non-sélectivité risquait d'être d'autant plus prononcée que l'organisation des chromosomes génomiques peut varier de façon importante d'une souche à l'autre, à l'instar de ce que l'on observe au niveau des propriétés immunologiques des légionelles. A ces difficultés s'ajoutent celles résultant de la non-sélectivité susceptible d'être observée en raison des hybridations parasites auxquelles peuvent donner lieu les ADN des autres espèces qui souvent accompagnent les légionelles dans les milieux qui les contiennent. C'est pourtant l'adaption de cette technique qui allait permettre la mise au point d'une technique propre, sélective et rapide de détection de L.P. dans tous les milieux susceptibles de les contenir, qu'il s'agisse de prélèvements pathologiques ou de prélèvements liquides ou atomosphériques, notamment des eaux, volumes d'eau ou d'air brassés dans toutes les installations impliquant des échanges thermiques entre des liquides et l'atmosphère extérieure (systèmes de refroidissement ou de chauffage), etc..

L'invention repose sur la découverte qui a été faite que, lorsque l'on utilise comme matière hybridable de sondes des génomes entiers de *Legionella pneumophila* fragmentés par l'enzymes de restriction *Bam*HI, préalablement débarrassés des fragments de restriction possédant des extrémités *Bam*HI (ci-après dénommés "fragments *Bam*HI" ayant des tailles de l'ordre de 20 kb) et de préférence encore des fragments *Bam*HI dont les tailles s'échelonnent de 9,5 à 30 kb environ, on obtenait une sonde permettant la détection pratiquement universalle des légionelles et plus particulièrement de *Legionella pneumophila*, cependant en même temps sélective en ce que la composition de fragments ainsi obtenus ne donne plus lieu à des réactions d'hybridation croisée avec des acides nucléiques des microorganismes qui accompagent souvent les légionelles dans les milieux d'où ils peuvent être isolés.

L'invention concerne par conséquent un procédé pour la fabrication d'une composition utile pour la détection des légionelles, plus particulièrement de *Legionella pneumophila*, procédé qui est caractérisé en ce que, partant de l'ADN génomique total d'une souche de *Legionella pneumophila*, on le fragmente par action de l'enzyme de restriction *Bam*HI, en ce que l'on sépare du mélange de fragments *Bam*HI obtenus principalement ceux qui ont des tailles de l'ordre de 20 kb, de préférence également ceux dont les tailles s'échelonnent entre 9,5 et 30 kb, et en ce que l'on recueille le mélange des autres fragments, ces fragments constituant alors la susdite composition utile pour la détection des légionelles. Cette composition est avantageusement marquée. Tout marqueur classiquement envisagé peut être utilisé. La composition peut être marquée à l'aide de traceurs radio-actifs. On peut également avoir recours à la modification des fragments d'ADN contenus dans cette composition par tout agent chimique permettant, notamment après hybridation avec des séquences d'ADN à rechercher, leur détection par des marqueurs enzymatiques, luminescents, fluorescents, etc..

Une composition préférée conforme à l'invention est obtenue à partir de la souche de L.P. que l'ont peut se procurer auprès de la Collection de Souches AMERICAN TYPE CULTURE COLLECTION sous le n° ATCC 33 152.

Des conditions analogues peuvent cependant être obtenues à partir d'un nombre de souches suffisamment élevé (notamment à partir des souches dont les numéros de dépôt à l'ATCC seront donnés plus loin) pour que l'on puisse en inférer raisonnablement qu'une composition analogue peut en fait être obtenue à partir de l'ADN génomique de toute souche de *Legionella pneumophila*.

L'invention concerne plus particulièrement encore les compositions elles-mêmes de fragments d'ADN génomique de *Legionella pneumophila*, utiles pour la détection sélective des légionelles, par hybridation avec les ADNs génomiques extraites de celles-ci ou simplement rendues accessibles à l'hybridation, ces compositions étant constituées d'un mélange de fragments d'ADN génomique de *Legionella pneumophila*, plus particulièrement caractérisé en ce qu'il est sensiblement exempt de fragments eux-mêmes hybridables à la fois, d'une part, avec des ADNs génomiques entiers de *Legionella pneumophila* et, d'autre part, avec des fragments *Bam*HI ayant des tailles d'environ 20 kb et obtenus à partir de génomes entiers de *Legionella pneumophila*, tels que des génomes entiers de la souche ATCC 33 152, par traitement de ces génomes entiers avec l'enzyme de restriction *Bam*HI et séparation ultérieure des fragments *Bam*HI ayant des tailles différentes.

Les fragments ayant une taille de l'ordre de 20 kb se sont révélés comme contenant apparemment des séquences qui s'hybrident de façon non sélective à des séquences ècomplémentaires portées aussi bien par des ADNs génomiques de légionelles que par des ADNs génomiques d'autres espèces et genres bactériens. Ceci étant, la sélectivité est encore davantage renforcée si l'on a soin de débarrasser également la composition de fragments selon l'invention des fragments *Bam*HI autres que ceux de 20 kb et dont les tailles s'échelonnent respectivement entre environ 9,5 et 30 kb.

En d'autres termes, des compositions préférées de l'invention sont formées de fragments *Bam*HI issus d'un génome, de préférence total de L.P., dont les tailles sont respectivement inférieures à environ 9,5 kb et supérieures à environ 30 kb.

La définition qui précède suppose qu'en fait le mélange de fragments résulte de façon pratiquement exclusive d'un traitement d'un génome entier de L.P. avec l'enzyme *Bam*HI, à l'exclusion de tout autre mode de fragmentation. Ceci étant, on peut envisager pour des raisons pratiques de soumettre le mélange à l'action d'autres enzymes de restriction, par exemple pour obtenir un mélange de fragments de tailles plus petites (pour ne pas parler des "fragmentations mécaniques" quelquefois difficilement évitables). On comprenda cependant aisément que les fragments plus petits issus de ces traitements, ayant des tailles comprises entre 9,5 kb et environ 30 kb résultant de la fragmentation supplémentaire de fragments *Bam*HI qui, antérieurement, avaient des tailles supérieures à 30 kb, pourront être aisément distingués des fragments *Bam*HI ayant des tailles comprises entre 9,5 et 30 kb, tels qu'ils ont été définis plus haut, en ce qu'ils ne forment pas d'hybrides mutuels dans les conditions appropriées, au moins dans des proportions substantielles.

Le cas échéant, on ne retiendra pour la réalisation des compositions de sondes qu'une partie des fragments issues de la mise en oeuvre du procédé sus-défini. Il reviendra chaque fois au spécialiste de preser l'un contre l'autre l'accroissement de sélectivité qui pourrait éventuellement résulter de la diminution du nombre de fragments constitutifs du mélange, contre la diminution de sensibilité qui pourra découler de l'élimination de certaines séquences particulièrement efficaces au niveau de l'hybridation, ne serait-ce qu'en raison de leur caractère hautement répétitif dans le génome des L.P.

Les susdites opérations de séparation de fragments *Bam*HI ou autres entre eux peuvent être réalisées de toutes façons en soi connues. Elles peuvent être réalisées par exemple par gel-filtration, electrophorèse, centrifugation dans des gradients appropriés, toutes ces méthodes ayant évidemment en commun la capacité de séparer des fragments d'ADN selon leurs tailles respectives.

L'invention concerne encore les procédés de détection eux-mêmes mettant en oeuvre des compositions conformes à l'invention, celles-ci ayant au préalable été marquées (par exemple radio-activement) ou rendues aptes à être détectées ou reconnues par des molécules affines ou aurtres produits, eux-mêmes marqués ou marquables, notamment après hydridation d'au moins certains des fragments de la composition avec les ADNs génomiques de L.P. à détecter.

On rappelle que les opérations essentielles s'attachant à une telle détection sont de préférence les suivantes:

dépôt et fixation des acides nucléiques des cellules à analyser (ou de ces dernières préablement traitées de façon à permettre l'accès à leurs acides nucléiques) sur un support approprié, tel que filtre de nitrocellulose, du polyacrylamide NYLON ou analogue;

mise en contact des acides nucléiques ainsi fixés ou rendus accessibles avec la composition de fragments d'ADN selon l'invention, dans des conditions permettant la réalisation d'une hybridation effective, lorsque des ADNs de légionelles sont présents;

élimination, notamment par lavage, de tout fragment d'ADN non hybridé;

détection des fragments marqués hybridés.

Comme on l'a dit plus haut, la détection peut mettre en jeu un traceur radio-actif qui avait auparavant été incorporé à la composition selon l'invention. Il pourra êtra préféré d'avoir recours à des marqueurs enzymatiques, luminescents ou fluorescents. Dans un tel cas, les fragements de la composition auront avantageusement été préalablement modifiés chimiquement pour y introduire des groupes chimiques ultérieurement couplables, directement ou indirectement, avec des molécules affines (notamment pour former des complexes) ou encore avec des anticorps capables de reconnaître ces groupes, ceux-ci présentant alors des propriétés d'haptène ou d'antigène. On pourra se reporter pour ce qui est des conditions dans lesquelles ces opérations peuvent être réalisées, par exemple au brevet français n° 78 10975 ou 81 24631 ou encore à la demande de brevet européen publiée n° 0063879.

D'autres caractéristiques de l'invention apparaîtront encore au cours de la description qui suit de réalisations pratiques de l'invention fournies à titre d'exemples non limitatifs et des conditions dans lesquelles l'invention peut être effectivement mise en oeuvre.

Le principe de la méthode selon l'invention repose sur la constatation qui a été faite de ce que les ADNs de L.P. préalablement marqués s'hybridaient de façon relativement sélective avec des ADNs de légionelles, et plus particulièrement encore de *Legionella pneumophila*. Cette sélectivité n'est cependant pas tout à fait suffisante. On constate en effet un certain niveau d'hybridation croisée entre les ADNs de L.P. et d'autres espèces qui souvent les accompagnent dans leurs milieux naturels. Certes les signaux d'hybridation sont plus faibles lorsque l'ADN hybridé avec un sonde constituée par un génome total de L.P. est constitué par un ADN provenant d'une auture espèce. Mais on comprendra que ces signaux même plus faibles peuvent fausser les mesures et surtout introduire des risques d'erreurs importants dans le cas de prélèvement contenant des proportions relatives importantes de bactériques étrangères par rapport au L.P. Parmi ces autres espèces, on mentionnera essentiellement les *Enterbacteriaceae,* les *Pseudomonadaceae,* les *Vibrionaceae,* et plus particulièrement encore les souches qui ont été étudiées dans la cadre des essais qui ont conduit à l'invention:

*Acinetobacter calcoaceticus*
*Chromobacterium violaceum*
*Pseudomonas aeruginosa*
*Aeromonas hydrophila*
*Serratia fonticola*
*Enterobacter intermedium*
*Escherichia coil*
*Alcaligenes faecalis,* etc..

Les essais peuvent être réalisés dans les conditions qui suivent:

1) *Obtention des ADNs à partir des bactéries étudiées*

L'ADN a été préparé selon la technique décrite par D. J. BRENNER et al (Journal of Clinical Microbiology, *15,* 1982).

2) *Fragmentation des acides nucléiques et récupération des fragments selon leurs tailles respectives*

Les ADNs purifiés obtenus dans les conditions qui précèdent ont été traités ensuite par diverses enzymes de restriction, plus particulièrement *Bam*HI et *Eco*RI, *Sma*I, *Xho*I, etc., selon les instructions de leurs fabricants respectifs. Après incubation pendant la nuit à la température ambiante (pour *Sma*I) ou à 37°C (pour les autres enzymes), on arrête la réaction par chauffage du milieu à 65°C pendant 10 minutes.

Deux méthodes ont été utilisées pour réaliser les séparations susdites.

a) *Séparation sur gradient de saccharose*

Les solutions de fragments d'ADN obtenues dans l'opération précédente sont soumises à une opération de séparation sur un gradient de saccharose 5—20%. Après centrifugation, on récolte les différentes fractions du gradient. Les tailles des fragments dans chaque fraction sont vérifiées en soumettant une aliquote de chaque fraction à une électrophorèse dans un gel d'agarose en présence de marqueurs de tailles (dans le cas présent ADN de phage lambda coupé par l'enzyme *Hind*III). Les fractions dont les fragments avaient les tailles inférieures à 9,6 kb ou supérieures à 30 kb ont été réunies et dialysées contre un tampon T.E. (Tris 10 mM, EDTA, 1 mM, pH 7,5) L'ADN a été précipité et redissous dans un volume minimum de TER.

b) *Séparation dans un gel d'agarose*

Les fragments sont séparées par électrophorèse dans un gel d'agarose. Les morceaux de gel correspondant aux différentes tailles de fragments à séparer sont alors découpés. Les ADN contenus dans les autres parties du gel sont alors extraits du gel par des techniques classiques, par exemple par la méthode dite de "compression sous congélation" (de l'anglais "freeze squeeze method") ou par électrophorèse dans un boudin à dialyse.

3) *Dépôt sur les filtres des échantillons à étudier*

Les hybridations ont été réalisées sur filtre de nitrocellulose (filtres BA85 de Schleicher et Schüll) ou sur filtres de NYLON (POSIDYNE NAZ der PALL).

Les dépôts des ADNs à tester sur les filtres ont été réalisés, essentiellement selon la technique décrite par Grünstein et Hogness. Les colonies sont déposées sur les filtres, lysées *in situ* avec une solution de soude 0,5 N/HaCl 2,5 N pendant 10 minutes à la température ambiante, puis neutralisées avec un solution d'acétate de sodium pH 5. Les filtres sont ensuite séchés à 80°C pendant 2 heures.

4) *Hybridation*

Les filtres ont ensuite été pré-traités ou "préhybrides" avec un solution de 100 microgrammes/ml d'ADN dénaturé de sperme de hareng à 67°C, pendant 1 heure dans un milieu 2 × NaCL/Cit/5 × FPG et pendant un heure dans un milieu 2 × NaCl/cit; 1 × FPG; KH$_2$PO$_4$ 25 mM, EDTA 2 mM] dodecylsulfate de sodium (SDS) à 0,5% en poids et sulfate de dextrane à 10% en poids. Dans ce qui précède:

1 × FPG représente un solution contenant 0,02% de l'agent de saturation dénommé "FICOLL 400"; 0,002% de polyvinylpyrrolidone 350; (pour éviter les absorptions parasites d'ADN) et 0,02% de glycine (pourcentages en poids):

NaCl/Cit représente un solution NaCl 0,15 M et citrate de sodium 0,015 M.

On ajoute ensuite la composition d'ADN radioactive servant de sonde (ayant une activité de l'ordre de 5 × 10$^7$ cpm/microgramme) préalablement dénaturée par traitement à 100°C pendant 5 minutes, suivi d'un refroidissement brusque. On laisse l'hybridation se faire ensuite pendant la nuit (environ 17 heures à 67°C). On lave ensuite les filtres plusieurs fois, la dernière à 67°C, chaque fois pendant 20 minutes dans une solution 0,1 SSS; SDS à 0,1%. Les filtres sont ensuite autoradiographiés sur écran d'intensification "KODAK X OMAT" en utilisant des films "KODAK X AR-5".

L'analyse systématique des fragments d'ADN obtenus à partir tant des légionelles, et plus particulièrement des *Legionella pneumophila* que des autres souches sus-mentionnées, a conduit à la constatation que l'élimination à pneumo phila titre principal des fragments *Bam*HI ayant des tailles de l'ordre de 20 kb, avait pour effet la suppression des hybridations croisées qui ont pu être observées entre les légionelles et les autres souches.

La sélectivité s'est manifestée d'une façon particulièrement efficace dans les analyses de l'espèce *Legionella pneumophila*. Comme il a été indiqué plus haut, il est particulièrement avantageux d'utiliser des compositions d'ADN fragmenté par l'enzyme de restriction *Bam*HI préalablement débarrassé des fragments ayant des tailles comprises entre 9,5 et 30 kb, et plus particulièrement des bandes (majeures) correspondant aux tailles 21,4 et 16,2 kb et aux bandes (mineures) 28, 12,8 et 10 kb.

On décrit ci-après un mode de mise en oeuvre préféré des procédés d'isolement et de détection des légionelles. L'échantillon d'eau à tester (1 litre généralement) est filtré à travers une membrane de polycarbonate d'une porosité de 0,2 micron. Après filtration, la membrane est posée, face filtrante vers le haut, sur un papier filtre imbibé d'un tampon acide (pH 2,2) pendant 10 minutes, puis épongé rapidement sur un papier filtre sec et posé sur un troisième papier filtre imbibé d'un tampon à pH neutre. Après neutralisation, le filtre est posé sur und boîte de Pétri contenant une milieu nutritif gélosé sélectif et incubé à 37°C pendant un temps variable (une nuit en général). Après ce traitement, qui a pour but de teur sélectivement les microorganismes qui ne sont pas des légionelles, les résidus se trouvant sur le filtre sont mis en suspension dans un petit volume d'eau distillée stérile (quelques millilitres) et cette suspension est centrifugée 10 minutes à 10 000 g dans une centrifugeuse de type EPPENDORF. Le surnageant est jeté, et les protéines présentes dans le culot sont extraites (traitement à la soude 0,5 N pendant 10 minutes, neutralisation avec de l'acide chlorhydrique et du tampon Tris, 3 extractions au phénol, 3 extractions à l'éther éthylique).

On obtient ainsi une solution claire contenant essentiellement des acides nucléiques.

Cette solution est répartie en autant d'aliquotes que l'on veut faire de tests. Après dénaturation de l'ADN par chauffage (100°C, 5 minutes, refroidissement rapide dans la glace), chaque aliquote est filtrée sur une membrane de nitrocellulose de 0,45 de porosité, à l'aide d'un appareil de filtration de type "Hybri-dot". Chaque aliquote donne une "tache" d'ADN d'environ 3 millimètres de diamètre. Le filtre est mis au four à 80°C pendant 2 heures et chaque tache est découpée, avec un perforateur ou emporte-pièce de diamètre approprié: par example 5—7 mm). Chacune des petites rondelles est hybridée avec une sonde conforme à l'invention. Après hybridation et séparation de l'excédent de sonde non fixée dans les conditions usuelles, on détecte directement sur le papier filtre la présence ou au contraire l'absence de sonde, soit par auto-radiographie, soit par toute autre méthode dans le cas où la sonde est marquée ou détectable par un marqueur non radio-actif, par exemple enzymatique.

On a fait référence ci-dessus à des brevets antérieurs dans lesquels des procédés de marquage non radio-actifs ont été envisagés. Il est particulièrement avantageux d'utiliser des sondes marquées par un N-acétoxy-N-2-acétylamino-fluorène (AAF) dont la fixation sur les compositions d'ADN peut être réalisée essentiellement selon les techniques décrites dans le brevet France n° 81 24631, qui a été identifié plus haut. Dans ce dernier cas, la présence de *Legionella pneumophila* se traduit par l'apparition d'une tache colorée après détection de la sonde "ADN—AAF", particulièrement lorsque l'on met en oeuvre des anticorps anti-AAF tels que décrits dns le brevet susmentionné, eux-mêmes étant ensuite reconnus par une seconde catégorie d'anticorps sur lesquels une enzyme est fixée qui présente la caractéristique de modifier la coloration d'un substrat spécifique.

Le procédé qui vient d'être décrit a permis la reconnaissance sélective, notamment des ADNs des souches suivantes (pour ne citer que des exemples):

*L. pneumophila* sérogroupe 1 — ATCC 33152,
*L. bozemanii* — ATCC 33217,
*L. longbeachae* sérogroupe 1 — ATCC 33462,
*L. micdadei* — ATCC 33204,
*L. wadworthi* — ATCC 33877,
*L. oakridgensis* — ATCC 33761,
*L. jordanis* — ATCC 33623,
*L. feelei* — ATCC 35072.

Le procédé ci-dessus est d'une grande sensibilité. Il permet le traitement de grands volumes liquides ayant des teneurs faibles en microorganismes. Ce procédé est particulièrement avantageux grâce à sa grande sensibilité et la rapidité de sa mise en oeuvre. En effet, il est possible de détecter moins de 300 picogrammes d'ADN de la bactérie recherchée, soit moins de 100,000 bactéries, au total. A titre de comparaison, les méthodes immunologiques actuelles nécessitent l'obtention de colonies visibles à l'oeil nu comptant plusieurs millions de bactéries, ce qui demande 4 à 6 jours de culture.

Au contraire, le procédé selon l'invention permet de détecter les légionelles en moins de 24 heures, dès que l'eau traitée compte plus de 100.000 bactéries par litre.

Il a encore été constaté que les fragments *Bam*HI retirés des compositions d'ADN conformes à l'invention avaient pour propriétés spécifiques de s'hybrider aux ARN ribosomaux des légionelles ou d'autres espèces, en particulier de *E. coli*, et plus particulièrement avec les ARNs 16 S et 23 S. De tels ARNs ribosomaux sont commercialisés par la société BOEHRINGER MANNHEIM (R.F.A.). La composition d'ADN selon l'invention peut donct encore être défini comme étant constituée par un mélange de fragments d'ADN génomiques de *Legionella pneumophila* essentiellement dépourvu des séquences d'ADN qui, dans les génomes, sont susceptibles d'être transcrites dans ces ARNs ribosomaux.

L'invention concerne donc encore en variante un procédé pour la préparation des compositions d'ADN selon l'invention, caractérisé en ce que l'on fragmente l'ADN génomique, total des L.P., avec une enzyme de restriction, notamment *Bam*HI, ou par tout autre moyen et en ce que l'on sépare ceux des fragments qui hybrident avec des RNA ribosomiques de *E. coli* ou de L.P. et en ce que l'on recueille les autres fragments non hybridés.

Pour hybrider cette opération, on aura avantageusement recours à des colonnes de chromatographie d'affinité mettant en oeuvre des agaroses activées, du type SEPHAROSE activée, par exemple celle commerecialisée sous la désignation SEPHAROSE 6MB. Il est connu que des acides nucléiques peuvent être fixés sur ces agaroses activées, notamment par contact de ces dernières avec une solution de l'acide nucléique à fixer au sein d'un tampon également recommandé par le fabricant desdites agaroses activées.

L'invention concerne plus généralement encore des sondes pour la détection sélective d'espèces et même de genres déterminés de bactéries. Ces sondes sont caractérisées en ce qu'elles sont constituées par des mélanges de fragments d'ADNs génomiques du genre bactérien considéré, ayant la capacité de s'hybrider avec des compositions d'ADNs originaires dudit genre ou avec les bactéries elles-mêmes, dès lors que les ADNs de celles-ci auront été rendues accessibles à l'hybridation, ce mélange étant cependant essentiellement exempt de fragments qui sont eux-mêmes hybridables, d'une part, avec des ADNs génomiques originaires des bactéries dudit genre et, d'autre part, avec des fragments d'ADNs également hybridables avec les ADNs génomiques susdits, mais hybridables également avec des ADNs provenant de bactéries d'un genre ou d'une espèce différent.

Des compositions préférées pour la détection sélective des bactéries du genre considéré sont exemptes des séquences de l'ADN génomiques susceptible d'être transcriptes en ARN ribosomal. Des compositions particulières préférées sont celles qui sont essentiellement exemptes des séquences d'ADN génomique qui hybrident avec les ARNs ribosomiques 16 S et ou 23 S de *E. coli.*

La possibilité d'utiliser à titre de sondes sélectives pour la détection es bactéries appartenant à un genre ou à une espèce déterminé, de compositions de fragments d'ADN contenant la majeure partie de l'ADN génomique des bactéries du genre ou de l'espèce considéré, à l'exclusion d'une proportion mineure de certaines séquences seulement, est un résultat tout à fait remarquable.

L'expression "mélange essentiellement exempt" n'exclut néanmoins pas que les compositions d'ADN selon l'invention puissent encore contenir certaines séquences, à la fois non répétitives et non sélectives, dès lors que ces dernières séquences n'interfèrent pas de façon essentielle avec la sensibilité et la sélectivité de la détection des séquences d'ADN caractéristiques du genre ou de l'espèce considéré, présentes en des proportions prédominantes. En particulier, le signal fourni par les séquences prédominantes est beaucoup plus fort que celui fourni par les séquences minoritaires non sélectives éventuellement encore présentes, si bien que la sécurité de la détection peut être assurée. Il va de soi que l'expression "signal" correspond à la nature du phénomène mis en oeuvre pour la détection sélective, par exemple la radioactivité, l'action sur un substrat enzymatique, etc. du marqueur porté par la composition utilisée à titre de sonde.

L'invention concerne encore un procédé pour préparer une composition d'ADN utilisable comme sonde spécifique pour la détection des bactéries d'un genre déterminé.

Ce procédé comprend essentiellement les étapes qui seront décrites ci-après. La matière première pour la préparation d'une telle composition consiste en l'ADN génomique total d'une bactérie appartenant au genre considéré. Cette bactérie correspond de préférence à celle dont la détection est recherchée. Par exemple, elle appartient à une espèce ou sous-espèce déterminée. Dans ce qui précède, cette bactérie, ci-après dénommée "bactérie de référence" appartenait à l'espèce *Legionella pneumophila*.

La première étape consiste dans l'isolement des ADNs appartenant à des genres de bactéries qui, dans la nature, accompagnent souvent les bactéries du genre à détecter. Des exemples de genres de bactéries que l'on rencontre souvent avec les légionelles ont été rappelées plus haut. On réalise alors des dépôts de ces ADNs génomiques totaux sur un support approprié, notamment de nitrocellulose ou de NYLON, dans des conditions classiques pour la réalisation d'opérations d'hybridation. On effectue ensuite des tentatives d'hybridation entre l'ADN génomique total obtenu à partir de la bactérie de référence avec les ADNs génomiques totaux appartenant aux autres genres retenus pour l'essai.

Dans la mesure où l'ADN génomique total contient des proportions substantielles de séquences non sélectives communes avec des séquences contenues dans certains au moins des ADNs génomiques totaux des autres bactéries, on observera une hybridation effective entre l'ADN génomique total de la bactérie de référence et de l'ADN génomique total de l'une au moins des autres bactéries, par exemple celle que l'on désignera ci-après, pour la commodité de l'exposé, par l'expression "bactérie 1".

La deuxième étape du procédé selon l'invention comprend alors la réalisation de digestions de préparations d'DN génomique total de la bactérie de référence, respectivement avec diverses enzymes de restriction, par exemple les enzymes *Bam*HI, *Eco*Ri, *Hind*III, *Hpa*I, *Pvu*II, *Sma*I, *Xho*I, etc. et, pour chacune des préparations, la séparation selon leurs tailles, des fragments d'ADN résultant de la digestion par l'enzyme de restriction correspondante, par exemple par électrophorèse des fragments sur un gel approprié et par le transfert des fragments séparés les uns des autres sur un support d'hybridation, par exemple par la méthode bien connue de SOUTHERN.

La deuxième étape du procédé consiste alors dans des essais d'hybridation des fragments ainsi isolés les unes des autres de chacune des préparations avec l'ADN génomique total de la "bactérie 1".

Les observations suivantes peuvent alors être faites au niveau de chacune des préparations d'ADNs génomiques de la bactérie de référence. Dans le cas de certaines préparations, on peut observer des hybridations impliquant un nombre important de fragments de tailles différentes présents sur le filtre d'hybridation. Dans le cas où l'ADN génomique de la "bactérie 1" avait été marqué radiactivement, on observe, à l'occasion de la détection photographique de la radioactivité retenue sur le filtre, des taches allongées, quelquefois désignées sous l'expression anglaise "smears" (trainées). Cette constatation traduit en général la présence de séquences hautement répétitives tant dans l'ADN génomique de la bactérie de référence que dans l'ADN génomique de la "bactérie 1". De telles constatations conduisent à la mise à l'écart de la préparation en cause.

Au contraire, l'observation pour d'autres préparations d'hybridations localisées seulement au niveau d'une ou d'un nombre limité des fractions d'ADN sur le filtre de nitrocellulose avec l'ADN de référence témoigne a contrario de la sélectivité des autres fractions séparées de l'ADN génomique de la bactérie de référence, puisque ces dernières fractions ne conduisent a aucune hybridation avec l'ADN de la "bactérie 1". C'est, par exemple, une telle observation qui a été faite, lorsque cette technique a été mise en oeuvre avec des ADNs de L.P., et sur une préparation qui avait été digrérée auparavant avec l'enzyme *Bam*HI.

Partant d'une préparation d'ADN génomique totale de la bactérie de référence, il suffit, pour obtenir un mélange d'ADNs de la bactérie de référence, qui ne donne plus lieu à une hybridation avec l'ADN génomique total de la "bactérie 1", de digérer l'ADN génomique total de la bactérie de référence avec la même enzyme de restriction (par exemple *Bam*HI dans le cas des L.P.), d'en éliminer les fragments présentant les tailles correspondant à celles des fragments qui, dans l'essai d'hybridation sus-indiqué, hybrident avec l'ADN de la "bactérie 1". On obtient ainsi un mélange de fragments d'ADNs de la bactérie de référence, qui permet d'ores et déjà la détection sélective de la bactérie de référence vis-à-vis de la "bactérie 1".

La troisième étape du procédé selon l'invention consiste alors à répéter l'essai d'hybridation de la première étape susdite du mélange de fragments d'ADNs génomiques originaires de la bactérie de référence, dépourvus des fragments hybridables avec l'ADN de la "bactérie 1", et obtenus au terme de la deuxième étape, avec l'ensemble des ADNs génomiques totaux des bactéries initialement mises en oeuvre dans le cadre de la première étape. L'observation éventuelle d'une nouvelle hybridation non sélective avec l'ADN génomique total, par exemple d'une "bactérie 2" conduira l'expérimentateur à répéter le cycle des opérations de sélection envisagée dans la deuxième étape sus-indiquée, mais en mettant cette fois-ci en oeuvre des essais d'hybridation entre l'ADN génomique total de la "bactérie 2" et des préparations de fragments d'ADN de tailles différentes, obtenues à partir de plusieurs préparations digérées par des enzymes de restriction respectivement distinctes, de l'ADN de la bactérie de référence. On peut ainsi obtenir à nouveau des mélanges d'ADNs issus de la bactérie de référence, sélectifs vis-à-vis de la "bactérie 2", après traitement de l'ADN génomique total avec l'enzyme de restriction appropriée de la bactérie de référence, et séparation de fragments hybridant avec l'ADN génomique de la "bactérie 2".

On peut observer que des séquences d'ADN communes à la bactérie de référence et à la "bactérie 1" coïncident au moins en partie avec des séquences communes à l'ADN total de la bactérie de référence et à l'ADN de la "bactérie 2". La séparation de la totalité de ces séquences communes à partir d'un ADN génomique total de la bactérie de référence conduit par conséquent à un mélange d'ADNs originaires de la bactérie de référence qui n'hybride plus, ni avec l'ADN de la "bactérie 1", ni avec l'ADN de la "bactérie 2".

En alternative, on peut obtenir une composition semblable n'hybridant plus avec les ADNs, tant de la "bactérie 1" que de la "bactérie 2", lorsque l'on utilise, dans les essais d'hybridation du second cycle susdit, le mélange de fragments obtenus à partir du génome total de la bactérie de référence, mais après séparation des séquences hybridant avec l'ADN de la "bactérie 1", en lieu et place du génome total.

Théoriquement, les opérations qui précèdent pourraient être répétées avec l'ADN génomique total d'une "bactérie 3". L'expérience montre cependant qu'en général ces expériences n'ont pas à être répétées plus de une ou deux fois. En effet, on ne perdra pas de vue que si des bactéries de genres distincts peuvent comporter une certain nombre de séquences communes, par exemple celles codant pour des ARNs ribosomaux, elles sont en général caractérisées par d'importantes séquences d'ADN qui leur sont tout à fait spécifiques. En outre, il s'agit surtout d'éliminer les séquences qui sont répétées souvent dans le génome bactérien. Des gènes isolés, éventuellement communs, ne peuvent pas en effet conduire à des signaux significatifs dans des essais d'hybridation sélectifs. Ils sont minoritaires en nombre vis-à-vis des séquences réellement sélectives, une fois que le mélange de ADNs qui les contiennent a été débarrassé desdites séquences répétitives. Et les séquences répétées sont peu nombreuses dans les bactéries. Elles sont constituées essentiellement des séquences codant pour des ARN ribosomiques et des transposons.

Dans ce qui précède, on a évoqué essentiellement l'application de ces techniques à la préparation de compositions utilisables comme sondes pour la détection sélective de bactéries appartenant à un genre déterminé. Il va de soi que cette technique peut être poussée plus loin si nécessaire, pour produire des préparations d'ADN qui permettent la détection sélective d'une espèce particulière de bactéries au sein d'un genre déterminé.

Les techniques qui viennent d'être décrites, et qui ont naturellement été mises en oeuvre pour la préparation de compositions d'ADN pour la détection sélective de L.P., sont susceptibles d'être mises en oeuvre dans des conditions tout à fait semblables pour la détection sélective de genres bactériens, voire d'espèces bactériennes totalement différentes. On mentionnera la possibilité d'appliquer ce procédé dans des conditions semblables à la détection sélective de bactéries appartenant à des groupes taxonomiques phylogénétiquement isolés, par exemple *Brucella, Campylobacter, Bordetella, Acinetobacter, Haemophylus ducreyi,* etc.

Dans une variante du procédé qui a été décrit plus haut, on peut également avoir recours, dans le cadre de la deuxième étape, à une hybridation des préparations fragmentées selon leurs tailles et transférées sur filtre, avec des ARNs ribosomaux des *E. coli,* par exemple ceux qui ont été identifiés plus haut. Après l'élimination, à partir d'une préparation d'ADNs digérés par une enzyme de restriction correspondante, de ceux des fragments qui hybrident avec ces ARNs ribosomaux, on obtient des préparations dont la sélectivité peut alors être testée à nouveau dans les conditions, qui ont été définies dans la troisième étape susdite. Le cas échéant, le procédé selon l'invention est alors répété dans les conditions décrites dans les première, deuxième et troisième étapes, si l'élimination des séquences hybridant avec les ARNs ribosomaux ne suffit pas à assurer l'obtention de préparations d'ADNs suffisamment sélectifs pour la détection pratiquement certaine de la bactérie du genre, voire de l'espèce considérée. On remarquera que des ARNs ribosomaux d'autres bactéries que *E. coli* peuvent être mise en oeuvre. Cependant on rappellera qu'il existe de grandes homologies entre les ARNs ribosomaux des différentes espéces bactériennes. Ces ARNs ribosomaux sont en effet caractérisés par une grande stabilite de séquence au cours de l'évolution phylogénétique (ou de ce qu'il est convenu d'appeler l'évolution des êtres vivants).

L'invention présente un caractère général vis-à-vis de l'ensemble des espèces bactériennes. Dans son principe, qui est d'éliminer les séquences pouvant présenter des hybridations croisées gênantes, elle peut être appliquée à la détection sélective d'autres micro-organismes, notamment de virus.

Par exemple, on obtient une composition utilisable comme sonde pour la détection des *Brucella*, en traitant une composition d'ADN de *Brucella* dans des conditions expérimentales semblables à celles décrites en rapport avec les *Legionella pneumophila*, mais en remplaçant l'enzyme *Bam*HI utilisée pour la restriction par l'enzyme *Kpn*I et en séparant de la composition de fragments obtenue les fragments *Kpn*I de 6,5 kb qui hybrident avec les ARNs ribosomiques tant des *Brucellae* que de *E. coli*, en particulier avec les ARNs 16S et 23S susmentionnés.

On a opéré notamment dans les conditions suivantes:

Partant d'une composition constituée d'ADNs de *Brucella melitensis* biovar 1, souche de référence "16 M" (BCCB R1; ATCC 23456), on la traite avec l'enzyme *Kpn*I et, après fragmentation, on sépare le fragment d'ADN de 6,5 kb, correspondant aux gènes condant pour les arns 16 et 23S, par centrifugation dans un gradient de saccharose ou par électrophorèse en agarose. La composition ensuite utilisée comme "composition sonde" est par conséquent constituée par l'ensemble des fragments d'ADN ayant des poids moléculaires soit inférieurs, soit supérieures à environ 6,6 kb, après marquage dans les conditions sus-indiquées. Dans le présent essai, la sonde a été marquée par translation de coupure ("nick-translation") avec $^{32}$P. La sonde a été jusqu'à présent testée dans des essais d'hybridation avec des ADNS de *Flavovacterium, Xanthomonas, Yersinia, Serratia, E. coli, Pseusomoniae Enterobacter, proteus, Salmonella, Citrobacter, Haquia, Actinobacillus, Pasteurella, Listeria, Haemophilus, Brucella, Acinetobacter, Megionella* et *Aeromonas,* déposés sur filtre nylon (Amersham et Pall) en taches ou "spots" de 0,3 µg. Des réactions d'hybridation n'ont été observées qu'avec les *Brucella*.

Comme il va de soi et comme il résulte d'ailleurs déjà de ce qui précède, l'invention ne se limite nullement à ceux de ses modes d'application et de réalisation qui ont été plus spécialement envisagés; elle en embrasse au contraire toutes les variantes.

**Revendications**

1. Composition pour l'élaboration d'une sonde en vue de la détection sélective d'espèces et même de genres déterminés de bactéries, caractérisée en ce qu'elle est constituée par un mélange de fragments d'ADN génomique de l'espèce ou du genre determine à détecter, cette composition étant cependant exempte des séquences de l'ADN génomique de l'espèce ou du genre considéré qui sont susceptibles d'être transcrites en ARN ribosomal, et qui sont communes avec celles d'autres espèces ou genres.

2. Composition selon la revendication 1, caractérisée en ce qu'elle est essentiellement exempte des séquences d'ADN génomique qui s'hybrident avec les ARNs ribosomiques 16 S et 23 S de *E. coli*.

3. Composition pour la détection sélective des légionelles, plus particulièrement de *Legionella pneumophila*, caractérisée en ce qu'elle est constituée par un mélange de fragments d'ADN génomique de *Legionella pneumophila* ayant la capacité de s'hybrider avec les compositions d'ADN originaires de légionelles, extraites de celles-ci ou rendues accessibles à l'hybridation, ce mélange étant cependant sensiblement exempt de fragments eux-mêmes hybridables à la fois, d'une part, avec des ADNs génomiques entiers de *Legionella pneumophila* et, d'autre part, avec des fragments *Bam*HI ayant des tailles d'environ 20 kb et obtenus à partir de génomes entiers de *Legionella pneumophila*, par traitement de ceux-ci par l'enzyme de restriction *Bam*HI et séparation des fragments *Bam*HI ayant d'autres tailles.

4. Composition selon la revendication 3, caractérisée en ce que pour augmenter la sélectivité, on élimine du susdit mélange de fragments, les fragments *Bam*HI ayant des tailles comprises entre environ 9,5 et 30 kb et obtenus à partir de génomes de *Legionella pneumophila*, par traitement de ceux-ci par l'enzyme de restriction *Bam*HI et après séparation des fragments *Bam*HI ayant des tailles inférieures à environ 9,5 kb et supérieures à environ 30 kb.

5. Composition selon la revendication 3 ou la revendication 4, caractérisée en ce qu'elle est exempte de fragments hybridables avec des fragments *Bam*HI ayant des tailles d'environ 20 kb, et notamment de fragments *Bam*HI ayant des tailles s'échelonnant d'environ 9,5 à environ 30 kb et obtenus à partir du mélange de fragments obtenus par traitement de la souche de L.P. ATCC 33152 avec l'enzyme *Bam*HI.

6. Composition selon l'une quelconques des revendications 3 à 5, caractérisée en ce que le susdit mélange est formé par des génomes entiers fragmentés de *Legionella pneumophilae*, sensiblement exempts des susdits fragments d'environ 20 kb.

7. Composition selon la revendication 6, caractérisée en ce que le susdit mélange de fragments est essentiellement exempt de fragments *Bam*HI ayant des tailles comprises entre environ 9,5 et environ 30 kb.

8. Composition selon la revendication 6 ou la revendication 7, caractérisée en ce qu'elle contient des fragments *Bam*HI ayant des tailles inférieures à environ 9,5 kb et supérieures à environ 30 kb.

9. Composition de fragments d'ADN génomique de *Legionella pneumophila* essentiellement exempte des séquences de l'ADN génomique qui sont susceptibles d'être transcrites en ARN ribosomal.

10. Composition selon la revendication 9, caractérisée en ce qu'elle est essentiellement exempte des séquences d'ADN génomiques qui hybrident avec les ARN ribosomiques 16 S et 23 S de *E. coli*.

11. Composition selon l'une quelconque des revendications 3 à 10, caractérisée en ce que les fragments sont marqués.

12. Composition pour la détection sélective des légionelles, plus particulièrement de *Brucella,* caractérisée en ce qu'elle est constituée par un mélange de fragments d'ADN génomique de *Brucella* ayant la capacité de s'hybrider avec les compositions d'ADN originaires de *Brucella,* extraites de celles-ci ou rendues accessibles à l'hybridation, ce mélange étant cependant sensiblement exempt de fragments eux-mêmes hybridables à la fois, d'une part, avec des ADNs génomiques entiers de *Brucella* et, d'autre part, avec des fragments *Kpn*I ayant des tailles d'environ 6,5 kb et obtenus à partir de génomes entiers de *Brucella,* par traitement de ceux-ci par l'enzyme de restriction *Kpn*I et séparation des fragments *Kpn*I ayant d'autres tailles.

13. Procédé pour produire une matière utilisable pour la production d'une sonde pour la détection sélective d'une espèce et même d'un genre déterminé de bactérie, caractérisé en ce que, partant de préférence de la totalité du génome entier, préablement isolé de bactéries appartenant à l'espèce ou même du genre déterminé susdit, on le fragmente avec une enzyme de restriction et on met en contact le mélange de fragments obtenu avec des ARNs ribosomaux de *E. coli,* on sépare les fragments d'ADN hybridés avec ces ARNs ribosmaux et l'on récupère le mélange des fragments d'ADN n'ayant pas conduit à une hybridation.

14. Procédé pour produire une matière utilisable pour la production d'une sonde pour la détection sélective des *Legionella pneumophilae,* caractérisée en ce que, partant de préférence de la totalité du génome entier préalablement isolé d'une souche de *Legionella pneumophila,* on le traite avec l'enzyme de restriction *Bam*HI dans des conditions permettant la fragmentation des acides nucléiques de ce génome en fragments *Bam*HI, en ce que l'on sépare les fragments *Bam*HI produits ayant une taille moléculaire de l'ordre de 20 kb et de préférence également l'ensemble des fragments *Bam*HI dont les tailles s'échelonnent de 9,5 à 30 kb, et en ce que l'on recueille les fragments restants qui constituent la susdite matière utilisable pour la production de sondes pour la détection des légionelles.

15. Procédé selon la revendication 14, caractérisé en ce que la totalité du génome de *Legionella pneumophila* initialement mis en oeuvre provient de la souche ATCC 33252.

16. Procédé pour produire une matière utilisable pour la production d'une sonde pour la détection sélective des *Brucella,* caractérisée en ce que, partant de préférence de la totalité du génome entier préablement isolé d'une souche de *Brucella,* on le traite avec l'enzyme de restriction *Kpn*I dans des conditions permettant la fragmentation des acides nucléiques de ce génome en fragments *Kpn*I, en ce que l'on sépare les fragments *Kpn*I produits ayant une taille moléculaire de l'ordre de 6,5 kb et en ce que l'on recueille les fragments restants qui constituent la susdite matière utilisable pour la production de sondes pour la détection des *Brucella.*

17. Composition selon la revendication 14 ou 15, caractérisé en ce que les acides nucléiques de la susdite matière sont marqués radio-activement ou par des marqueurs, notamment enzymatiques, luminescents ou fluorescents.

18. Procédé de détection de *Legionella pneumophila* caractérisé par la mise en contact de l'ADN d'une souche bactérienne ou d'un mélange de souches bactériennes à analyser avec un composition selon l'une quelconque des revendications 3 à 11, dans des conditions autorisant une hybridation éventuelle et par la détection des hybrides éventuellement formés.

**Patentansprüche**

1. Zusammensetzung für die Herstellung einer Sonde für die spezifische Bestimmung der Art oder Gattung einer Menge von Bakterien, dadurch gekennzeichnet, daß sie gebildet ist aus einer Mischung von genomischen DNA-Fragmenten der zu bestimmenden Art oder Gattung, wobei diese Mischung frei ist von solchen genomischen DNA-Sequenzen der Art oder Gattung, die zu ribosomer RNA transcribierbar sind, und die andere Arten oder Gattungen ebenfalls aufweisen.

2. Zusammensetzung nach Anspruch 1, dadurch gekennzeichnet, daß sie im wesentlichen frei ist von solchen genomischen DNA-Sequenzen, die mit ribosomischen Ribonucleinsäuren 16 S und 23 S von E. *coli* hybridisieren.

3. Zusammensetzung für die selektive Bestimmungen von Legionellen, insbesondere von *Legionella pneumophila,* dadurch gekennzeichnet, daß sie gebildet ist aus einer Mischung von genomischen DNA-Fragmenten von *Legionella pneumophila,* die die Fähigkeit besitzen mit von Legionellan stammenden DNA-Mischungen zu hybridisieren, wobei diese aus solchen gewonnen sind, die einer Hybridisierung zugänglich sind oder zugänglich gemacht wurden, und wobei diese Mischung deutlich frei ist von Fragmenten, die gleichzeitig hybridisierbar sind einerseits mit vollständigen genomischen DNA's von *Legionella pneumophila* und andererseits mit *Bam*HI-Fragmenten einer Länge von etwa 20 kb, die erhalten werden, ausgehend von vollständigen Genomen von *Legionella pneumophila* durch ihre Behandlung mit dem Restrictions-enzym *Bam*HI und Abtrennung derjenigen *Bam*HI-Fragmente mit anderen Größen.

4. Zusammensetzung nach Anspruch 3, dadurch gekennzeichnet, daß man zwecks Erhöhung der Selektivität von der Fragmentenischung solche *Bam*HI-Fragmente abrennt, die Größen zwischen etwa 9,5 und 30 kb aufweisen und die erhalten sind ausgehend von *Legionella pneumophila* durch deren Behandlung mit dem Restrictionsenzym *Bam*HI und die nach der Abtrennung der *Bam*HI-Fragmente Größen kleiner etwa 9,5 und größer etwa 30 kb aufweisen.

5. Zusammensetzung nach Anspruch 3 oder 4, dadurch gekennzeichnet, daß sie frei ist von Fragmenten, welche mit *Bam*HI-Fragmenten der Größe von etwa 20 kb hybridisieren, insbeson-

dere von solchen *BamHI*-Fragmenten der Größe zwischen etwa 9,6 und etwa 30 kb, und die erhalten wurden aus einer Mischung von Fragmenten, die gewonnen wurden durch Behandlung des Stammes L.P. ATCC 33 152 mit dem Enzym *BamHI*.

6. Zusammensetzung nach einem der Ansprüche 3 bis 5, dadurch gekennzeichnet, daß die Mischung gebildet wird aus vollständigen fragmentierten Genomen von *Legionella pneumophila*, die deutlich frei sind von den besagten Fragmenten der Größe etwa 20 kb.

7. Zusammensetzung nach Anspruch 6, dadurch gekennzeichnet, daß die Fragmentenmischung im wesentlichen frei ist von *BamHI*-Fragmenten der Größe zwischen etwa 9,5 und etwa 30 kb.

8. Zusammensetzung nach Anspruch 6 oder 7, dadurch gekennzeichnet, daß sie *BamHI*-Fragmente der Größe unter etwa 9,5 und über 30 kb enthält.

9. Zusammensetzung von genomischen DNA-Fragmenten von *Legionella pneumophila*, dadurch gekennzeichnet, daß sie im wesentlichen frei ist von solchen genomischen DNA-Sequenzen, die zu ribosomer RNA transcribierbar sind.

10. Zusammensetzung nach Anspruch 9, dadurch gekennzeichnet, daß sei im wesentlichen frei ist von genomischen DNA-Sequenzen, die mit ribosomischen Ribonuleinsäuren 16 S und 23 S von *E. coli* hybridisieren.

11. Zusammensetzung nach einem der Ansprüche 3 bis 10, dadurch gekennzeichnet, daß die Fragmente markiert sind.

12. Zusammensetzung für die selektive Bestimmung von Legionellen, insbesondere von *Brucella*, dadurch gekennzeichnet, daß sie gebildet ist aus einer Mischung von genomischen DNA-Fragmenten von *Brucella* mit der Fähigkeit mit von *Brucella* stammenden DNA-Mischungen zu hybridisieren, die von diesen gewonnen sind oder der Hybridisierung zugänglich gemacht sind, wobei diese Mischung deutlich frei ist von solchen Fragmenten, die gleichzeitig hybridisierbar sind einerseits mit vollständigen genomischen DNA von *Brucella* und andererseits mit *KpnI*-Fragmenten der Größe von etwa 6,5 kb und die gewonnen wurden ausgehend von vollständigen Genomen von *Brucella* durch deren Behandlung mit dem Restrictionsenzym *KpnI* und Abtrennung der *KpnI* Fragmente mit anderen Größen.

13. Verfahren zur Herstellung einer Substanz, die geeignet ist zur Erzeugung einer Sonde für die selektive Bestimmung einer Gattung oder einer Art einer Menge von Bakterien, dadurch gekennzeichnet, daß man ausgehend vorzugsweise von der Gesamtheit des vollständigen Genoms, das zuvor aus Bakterien isoliert wurde, die zu der oben genannten Gattung oder Art gehören, dieses mit einem Restrictionsenzym fragmentiert und in Kontakt bringt mit einer Fragmentenmischung, die erhalten wurde mit ribosomischen RNA's von *E. coli*, danach die hybridisierten DNA-Fragmente mit diesen ribosomischen RNA's abtrennt und die Mischung von DNA-Fragmenten wiedergewinnt, die keine Hybridisierung eingegangen ist.

14. Verfahren, zur Herstellung einer Substanz, die geeignet ist zur Erzeugung einer Sonde für die selektive Bestimmung von *Legionella pneumophila*, dadurch gekennzeichnet, daß man ausgehend vorzugsweise von der Gesamtheit des vollständigen Genoms, das vorher von einem *Legionella pneumophila*-Stamm isoliert wurde, dieses mit *BamHI*-Restrictionsenzym unter Bedingungen behandelt, die die Fragmentierung von Nucleinsäuren des Genoms in *BamHI*-Fragmente gestattet, wobei man die *BamHI*-Fragmentprodukte abtrennt, die eine Molekulargröße von etwa 20 kb haben und vorzugsweise in gleicher Weise die Gesamtheit derjenigen *BamHI*-Fragmente, deren Größe von 9,5 bis 30 kb beträgt abtrennt, und wobei man die verbleibenden Fragmente gewinnt, die die besagte Substanz bilden, die zur Herstellung, von Sonden für die Bestimmung von Legionellen verwendbar ist.

15. Verfahren nach Anspruch 14, dadurch gekennzeichnet, daß die ursprünglich eingesetzte Gesamtheit des Genoms von *Legionella pneumophila* von Stamm ATCC 33252 gewonnen wurde.

16. Verfahren zur Herstellung einer Substanz, die für die Herstellung einer Sonde zur selektiven Bestimmung von *Brucella* geeignet ist, dadurch gekennzeichnet, daß man vorzugsweise ausgeht von der Gesamtheit des vollständigen, zuvor von einem *Brucella*stamm isolierten Genoms, wobei man dieses mit dem Restrictionsenzym *KpnI* unter Bedingungen behandelt, die die Fragmentierung von Nucleinsäuren dieses Genoms in *KpnI*-Fragmente erlaubt, die *KpnI*-Fragmentprodukte abtrennt, die eine Molekulargröße von etwa, 6,5 kb aufweisen und daß man die verbleibenden Fragmente gewinnt, die die genannte Substanz für die herstellung von Sonden für die Bestimmung von *Brucella* bilden.

17. Verfahren nach Anspruch 14 oder 15, dadurch gekennzeichnet, daß die Nucleinsäuren der genannten Substanz radioaktiv oder durch insbesondere enzymatische lumineszente oder fluoreszente Markierungsverbindungen markiert werden.

18. Verfahren zur Bestimmung von *Legionella pneumophila*, gekennzeichnet durch das In-Kontakt-Bringen von DNA eines Bakterienstammes oder einer Mischung von Bakterienstämmen zur Analyse mit einer Zusammensetzung gemäß einem der Ansprüche 3 bis 11 unter Bedingungen, die eine Hybridisierung erlauben und durch die Bestimmung der gegebenenfalls gebildeten Hybride.

**Claims**

1. A composition for elaborating a probe for the selective detection of species and even specified genuses of bacteria, wherein it is constituted of a mixture of fragments of genomic DNA of the specific species or genus to be detected, this composition however being exempt of those sequences of genomic DNA of the species or genus under consideration, which are susceptible to being transcribed in to ribosomal RNA, and which are common with those other genus or species.

2. The composition according to Claim 1, wherein it is essentially exept of genomic sequences which hybridize with the 16S and 23S ribosomal RNAs of *E. coli*.

3. A composition for the specific detection of legionellas, more particularly *Legionella pneumophila*, wherein it is constituted of a mixture of genomic DNA fragments from *Legionella pneumophila* having the capacity to hybridize with compositions of DNA originating from legionellas, being either extracted from them or rendered accessible to hybridization, this mixture however being essentially exempt of fragments which are themselves hybridizable on the one hand with genomic DNAs of *Legionella pneumophila*, and on the other hand with the *Bam*HI fragments having sizes of around 20 kb and obtained from whole genomes of *Legionella pneumophila* by treatment of them by the restriction enzyme *Bam*HI and separation from *Bam*HI fragments having other sizes.

4. The composition according to Claim 3, wherein for increasing selectivity *Bam*HI fragments having sizes between around 9.5 and 30 kb are eliminated from said mixture of fragments and obtained from *Legionella pneumophila* genomes by treatment of them by the restriction enzyme *Bam*HI and after separation from *Bam*HI fragments having sizes less than around 9.5 kb and more than around 30 kb.

5. The composition according to Claim 3 or Claim 4, wherein it is exempt of fragments hybridizable with *Bam*HI fragments having sizes of around 20 kb, and notably *Bam*HI fragments having sizes ranging from around 9.5 to around 30 kb and obtained from a mixture of fragments obtained by treatment of the L.P. strain ATCC 33152 with the enzyme *Bam*HI.

6. The composition according to any one of claims 3 to 5, wherein the aforesaid mixture is formed of fragmented whole genomes of *Legionella pneumophila*, essentially exempt of the aforesaid fragments of around 20 kb.

7. The composition according to Claim 6, wherein the aforesaid mixture of fragments is essentially exempt of *Bam*HI fragments having sizes between around 9.5 and around 30 kb.

8. The composition according to Claim 6 or Claim 7, wherein it contains *Bam*HI fragments having sizes less than around 9.5 kb and more than around 30 kb.

9. A composition of fragments of genomic DNA from *Legionella pneumophila* essentially exempt of genomic DNA sequences which are susceptible to be transcribed to ribosomal RNA.

10. The composition according to Claim 9, wherein it is essentially exempt of genomic DNA sequences which hybridize with the 16 S and 23 S ribosomal RNAs of *E. coli*.

11. The composition according to any one of claims 3 to 10, wherein the fragments are labeled.

12. The composition for the selective detection of legionellas, more particularly of *Brucella*, wherein it is constituted of a mixture of genomic DNA fragments from *Brucella* having the capacity to hybridize with DNA compositions originating from *Brucella*, either extracted from them or rendered accessible to hybridization, this mixture however being essentially exempt of fragments which are themselves hybridizable with both whole genomic DNAs of *Brucella* and with those *Kpn*I fragments having sizes of around 6.5 kb and obtained from whole genomes of *Brucella* by treatment of them by the restriction enzyme *Kpn*I and separation from *Kpn*I fragments having other sizes.

13. A process to produce a material utilisable for the production of a probe for the selective detection of a species and even a specified genus of bacteria, whereby, starting from preferably the totality of the whole genome, first isolated from bacteria belonging to the aforesaid species or even specified genus, the DNA is fragmented by a restriction enzyme and the mixture of fragments obtained are placed in contact with ribosomal RNAs from *E. coli*, those DNA fragments having hybridized with these ribosomal RNAs are separated out, and the remaining mixture of DHA fragments not having been hybridized are recovered.

14. A process for producing a material utilizable for the production of a probe for the selective detection of *Legionella pneumophila*, whereby starting preferably from the totality of the whole genome, previously isolated, of a strain of *Legionella pneumophila*, it is treated with the restriction enzyme *Bam*HI, whereby the *Bam*HI fragments produced having a molecular size of the order of 20 kb and preferably equally the ensemble of *Bam*HI fragments whose sizes range from 9.5 to 30 kb are separated out, and whereby the remaining fragments are collected which constitute the aforesaid material utilizable for the production of probes for the detection of legionellas.

15. The process according to Claim 14, whereby the totality of the genome of *Legionella pneumophila* initially used comes from the strain ATCC 33252.

16. A process for the production of a material utilizable for the production of a probe for the selective detection of *Brucella*, whereby starting preferably from the totality of the whole genome, previously isolated, of a strain of *Brucella*, it is treated with the restriction enzyme *Kpn*I under conditions permitting the fragmentation of the nucleic acids of the genome into *Kpn*I fragments, whereby the *Kpn*I fragments produced having a molecular size of the order of 6.5 kb are separated out, and whereby the remaining fragments are collected, which constitute the aforesaid material utilizable for the production of probes for the detection of *Brucella*.

17. The process according to Claim 14 or 15, whereby the nucleic acids of the aforesaid material are labeled radioactively or by enzymatic, luminescent or fluorescent labels.

18. A process for detection of *Legionella pneumophila* whereby placing into contact of the DNA of bacterial strain or a mixture of bacterial strains to be analyzed, with a composition according to any of the Claims 3 to 11, under conditions permitting a possible hybridization and by the detection of the hybrids possibly formed.